# EUROPEAN PATENT SPECIFICATION

(11) **EP 4 284 233 B1**
(45) Date of publication and mention of the grant of the patent: **22.04.2026**
(21) Application number: 22703959.1
(22) Date of filing: 28.01.2022
(51) Int. Cl.: A61B 5/103, A61B 5/11

(54) **A PRESSURE MONITORING DEVICE, SYSTEM AND METHOD**
EIN DRUCKÜBERWACHUNGSGERÄT, -SYSTEM UND -VERFAHREN
DISPOSITIF, SYSTEME ET PROCEDE DE SURVEILLANCE DE LA PRESSION

(30) Priority: 28.01.2021 GB 202101244
(43) Date of publication of application: 06.12.2023
(73) Proprietor: The Helping Hand Company (Ledbury) Limited, Herefordshire HR8 1NS (GB)
(72) Inventor: MOORE, Stuart, Hereford and Worcester HR8 1NS (GB)
(74) Representative: Maidment, Marc
(86) International application number: PCT/GB2022/050242
(87) International publication number: WO 2022/162389

(56) References cited:
- EP-A1- 3 323 342
- EP-A2- 2 298 264
- WO-A1-2014/186894
- WO-A1-2016/185364
- WO-A1-2020/039096
- WO-A1-2020/264140
- GB-A- 2 547 436
- US-A1- 2013 090 571
- US-A1- 2014 005 502

## Description

The present invention relates to a pressure monitoring device and particularly but not exclusively to a pressure monitoring device for monitoring the activity of a person on a seat such as a cushion, mattress or other structure for supporting the person in a seated or reclined position. The present invention also relates to a system using the pressure monitoring device and a method of managing pressure sore risk.

### BACKGROUND TO THE INVENTION

Patients, or individuals, who spend long periods of time in a single position, whether in hospital or otherwise, are at risk of pressure ulcers forming. Whilst the skin can tolerate a high pressure for short periods, or sustained lower pressure over a longer period, spending excessive time in one position can cause capillaries in the skin to close up, which may lead to skin necrosis and the subsequent formation of a pressure ulcer (also known as pressure sores or bedsores). Patients at risk of developing such ulcers include, for example, those who are elderly, paraplegic, tetraplegic, critically ill or terminally ill. The most common affected parts of the body can include the sacrum, the coccyx, the heels and the hips, although they may also form elsewhere.

Pressure ulcers can arise due to sustained pressure on an area of soft tissue, often over a bony prominence, with a range of factors potentially contributing to their formation, including but not limited to: shearing (where the skin remains static but the underlying tissues do not) and microclimate (temperature and level of moisture) between the bed and a patient's skin. Perspiration and incontinence both contribute to the skin microclimate, increasing the level of moisture and potentially accentuating the development of one or more pressure ulcers.

It can take many weeks for a pressure ulcer to completely heal, with more advanced ulcers potentially never fully healing, although the age and general state of health of the patient will also affect the outcome. They are painful at all stages for all patients and increase the likelihood of infection for the immunocompromised.

The early identification of pressure ulcers can greatly increase the chance of successful treatment, and although the technique of regularly turning bedridden patients has long been known to help reduce their prevalence, this requires nursing staff, for example, to adhere to a regular schedule of turning multiple patients.

Currently, some systems use pressure sensors to detect whether a person is sat on a seat or in bed, for example, which can further be used to identify if they have left the seat or bed, or possibly fallen off.

Other systems can only selectively measure the pressure under a person on a seat or bed, essentially creating a snapshot of the pressure at that point in time. This can be useful to determine if a particular seated position of the patient creates a higher risk of a pressure sore developing, due to excessive pressure points, determine where pressure points may occur.

Mitigating the risk of pressure sore formation generally relies on providing a suitable cushion to support the patient when seated and developing cushions which better distribute the pressure during use. Of course, a patient's weight can change or fluctuate and this may affect cushion suitability. This can result in the initially prescribed cushion becoming less effective over time. It is also impossible to ensure that the patient uses the cushion correctly, or indeed that they use the cushion at all, so the impact on pressure sore risk and/or management for a given patient has inherent uncertainty.

Document GB2547436 discloses a pressure-monitoring cushion incorporating two pressure-threshold sensors implemented as conductive sheets separated by resilient insulating layers. The first sensor indicates normal pressure, while the second, with higher threshold, indicates excessive pressure. A controller resets a timer when pressure redistributes and issues alerts if the occupant remains seated without redistribution for too long. Wireless alert signalling to an external device is included. WO2016/185364 discloses a pressure-sensitive pad placed under a mattress, using variably resistive layers whose resistance decreases under pressure. The pad may be divided into multiple zones for detecting presence, movement, weight, and position. A controller performs dynamic calibration, distinguishing mattress weight from occupant weight, and identifies activation/deactivation/transition zones.

It is an object of the present invention to reduce or substantially obviate the aforementioned problems.

### STATEMENT OF INVENTION

According to a first aspect of the present invention there is provided a pressure monitoring device as set out in claim 1. The device is for clinical use in monitoring the activity of a person on a seat such as a cushion, mattress or other structure for supporting the person in a seated or reclined position. The device comprises:
a pressure sensing means for sensing pressure exerted by the person, the pressure sensing means being configured to sense: pressure at or below, or up to, a first pressure threshold for corresponding to the absence of the person; pressure above the first pressure threshold and at or below, or up to, a second pressure threshold for corresponding to the seated or reclined person generating acceptable pressure; and pressure above the second pressure threshold for corresponding to the seated or reclined person generating excessive pressure;
a data logger connected to the pressure sensing means and configured to monitor the pressure sensing means, in which the data logger is configured to record pressure data over a continuous period of time to record each time the pressure applied to the cushion drops below or exceeds the first pressure threshold and each time the pressure applied to the cushion drops below or exceeds the second pressure threshold; and
a communication means connected to the data logger for selectively establishing a connection to an external electronic device and transmitting recorded data.

The disclosed pressure monitoring device provides a self-contained unobtrusive way of sensing and storing pressure data, which can be useful when a person is at home or in a health care setting, such as a care home, hospital ward, or similar. The term "clinical use" is intended to mean use in a variety of healthcare scenarios, including but not limited to the foregoing. According to the invention, the device is a cushion or a mat, for example, or a cover for a cushion.

Data can be recorded over a significant period of time (hours or days, or longer) without the need for a carer, clinician, therapist or similar needing to be present. A thorough, on-going understanding of the suitability of the seat such as a cushion, mattress or other structure for pressure care and its usage pattern by a patient over a period can be achieved. In particular, it is possible to review whether the seat such as a cushion, mattress or other structure is providing adequate support throughout a day of use.

This can assist in planning daily routines for the patient, or cushion user, and highlight any issues early on before they become serious. In other words, the device enables a preventative approach where a carer can proactively manage care of the patient in respect of pressure sores, rather than acting reactively once sores develop or fail to heal in the expected timeframe. It may also assist in identifying aspects of the person's daily routine which may potentially be problematic in respect of pressure sore development or treatment.

A clinician can access or retrieve the data by an external device. Similarly, the person using the cushion may access or retrieve the data by an external device, for example by means of an app on a tablet or smartphone. This can enable better self-monitoring and self-management of one's own risk in respect of pressure sores.

The pressure sensing means may include first and second pressure switches for the respective pressure thresholds. The pressure sensing means detects applied pressure on the seat such as a cushion, mattress or structure, rather than ambient or atmospheric pressure. One or more additional pressure switches may be provided for additional pressure thresholds. For example, a third pressure switch for a third pressure threshold may be provided. This can allow the device to distinguish between periods where pressure is somewhat higher than acceptable, and periods where pressure is much higher than acceptable. It is envisaged that the pressure switches may be part of a single pressure switch with multiple pressure thresholds, or set of pressure switches with multiple pressure thresholds.

Using a series of two or more pressure switches allows the device to record when specific pressure thresholds are exceeded (both when the pressure increases or decreases). This can provide further insight into the usage of the cushion over time, to enable a better understanding of pressure sore management for a given person.

It will be appreciated that using pressure switches can provide a general indication of whether pressure is low (or absent), medium (or acceptable) or high (or excessive). This can provide a low-cost way to assess pressure experienced by a person when sat down.

First and second pressure sensor units may be provided.

The first and second pressure sensor units may be horizontally spaced apart for occupying different positions underneath a seated or reclined person.

The pressure sensing means may be provided on top of a seat, within the body of a seat, or under a seat (i.e. on, within or under a cushioned portion that a user sits on). The communication means may be one-way for transmitting data to the external device, but is preferably two-way. The communication means may be a wireless communication means. The wireless communication means may be a short-range wireless communication means. For example, the communication means may operate using a Bluetooth^{®} connection, an infra-red connection or near-field communication (NFC). NFC may be preferred for wireless communication.

According to the invention, the device is a pressure monitoring cushion. The term "cushion" may include any device suitable for providing support to a person when seated or reclined, whether that is a cushion, seat squab, mattress or similar.

The device may be a cover for retrofitting to a cushion. The cover may be releasably engageable with the cushion.

The cushion may include any suitable support medium. For example, a support foam may be provided. The foam may have a thickness, firmness and/or density which is prescribed according to the person for whom the cushion is intended. The foam may provide a fixed or predetermined amount of support. This may be determined after assessment by a clinician, for example after using a 'prescriber' cushion such as that described in the Applicant's granted European patent EP3416603. The pressure sensing means and data logger do not change the support characteristics of the foam during use or between uses of the cushion. Rather the data can later be reviewed by a clinician or other carer and used to inform clinical decisions about whether providing an alternative cushion to provide a different amount of support is warranted.

A recess or cavity may be provided in the cushion. The recess/cavity may be formed by a cut-out provided in a portion of a body of the cushion (such as a portion of a support foam, for example).

The terms recess, cavity and cut-out are intended to be substantially interchangeable in this specification, and all refer to a space suitable for the data logging device and communication means. Whilst "cut-out" may mean that material has been actively cut out of the cushion to create the space, the term "cut-out" is not to be solely interpreted in this manner and "cut-out" may also mean that a space is formed in the cushion without actually requiring any cutting to create the space.

The cut-out may be provided in a lower surface of the body of the cushion, that is to say the surface opposite the surface on which a person sits or reclines, i.e. the upper portion. The cut-out or recess may be provided in a middle of the cushion, substantially equidistant between the upper and lower surfaces of the cushion. This is preferred where the cushion is intended to be usable either way up.

The cut-out may be provided in a corner of the cushion, preferably in a corner opposite the front of the cushion. The data logger and communication means may be disposed in the cut-out portion.

A housing may be provided to enclose the data logger and communication means. The housing may be provided in the cut-out, or sized to fit within the cut-out.

A cut-out, especially when spaced away from the upper surface and towards a rear corner, provides a way of protecting the data logger and communication means from accidental damage, and moreover reduces or removes possible sources of discomfort.

The pressure sensing means may be disposed on a lower surface of the body of the cushion. The pressure sensing means may be disposed within the body of the cushion.

The pressure sensing means may comprise at least one pressure sensor. The pressure sensing means may include a pair of pressure sensors for respective halves of the cushion (typically left- and right-hand sides). The pressure sensing means may include a plurality of pressure sensors, for example at least four pressure sensors for monitoring pressure in four respective quadrants underneath the user.

There may be concave areas in the cushion for receiving the buttocks and legs of a person. Where a pair of pressure sensors or switches are provided, they may be arranged in parallel, extending lengthwise underneath the left-hand side concave areas and the right-hand side concave areas respectively. If it is only necessary to measure pressure on one side (i.e. only on the left or only on the right), then only one of the pressure sensors/switches may be present, and may be located under the concave areas of that side.

By disposing the pressure sensing means away from the upper surface on which a person sits or reclines, it is possible to reduce or remove a source of discomfort.

An identifier or indicator adapted to identify the device from a plurality of substantially similar devices may be provided. The indicator may be a sounder. The sounder may be controllable by an external device through the communication means. The sounder may be provided in the housing.

An indicator is particularly useful as it provides a means to identify a particular device when there are a number of devices around.

The pressure data logger may be considered to be part of a use monitor to monitoring the use of a seat such as a cushion, mattress or other structure. The data logger should be configured to record pressure data for the pressure exerted on the seat, cushion, mattress or other structure by a person seated or reclined on the seat, cushion, mattress or other structure. The data logger should also record pressure data when the seat, cushion, mattress or other structure doesn't have a person seated or reclined on top of it, but the actual pressure value in that case is not important.

The data logger may also record time data.

The data logger is intended to record and accumulate data over a period of time, and to then transmit or allow access to the recorded data for evaluating the periods of use and non-use. Having a complete record of whether the seat, cushion, mattress or other structure has been used or not, and the pressure experienced during such use, is relevant for a clinical decision about the therapeutic benefit to a person.

An alert means may be provided for alerting the seated or reclined person to shift their position. A timer for timing a period of use may be provided. The timer may be configured to run for a set time period beginning when pressure data indicates that a person has just sat down or reclined on the cushion, mattress or other structure. The alert means may be configured to initiate an alert when the time period of the timer has elapsed. The alert means may produce an audible alert and/or a visual alert.

The alert means and timer provides a way of reminding the device user that they need to shift their position.

The set time period may dynamically change based on the sensed pressure. The set time period may be reduced based on the sensed pressure exceeding the second pressure threshold.

The alert may be produced if the second pressure threshold has been met or exceeded, such as for a minimum first time period. The alert may be produced if the pressure data indicates that the first pressure threshold has been met or exceeded for a minimum second time period. The first time period may be shorter than the second time period. For example, the first time period may be between 5 to 20 minutes and the second period may be in at least 20 minutes. The time periods are preferably set so as to minimise development of pressure ulcers.

The set time period of the timer may be set by a user of the external device, such as a clinician, carer or therapist.

The device may be battery-powered. To conserve battery power and prolong the period between battery replacement or recharge, one or more of the data logger, communication means and pressure sensing means may be configured to enter a low power or 'sleep' mode during use. However, the data logger and pressure sensing means should be configured to 'wake' the device and ensure data is recorded when there is a change in pressure as discussed above. For example, a vibration sensor or tilt switch may be used as a trigger for waking the system for example, as a person getting up from or sitting down on the cushion is liable to rock the cushion substantially.

The pressure monitoring device may be configured to identify changes in the pressure data which correspond to a change of position of the person during use. The pressure monitoring device may be configured to record one or more instances of the change of position. The pressure monitoring device may be configured to transmit one or more instances of the change of position for providing information on the person's change in position on the seat to the external electronic device.

For example, a change in pressure of more than ±5% or ±10%, relative to an immediately preceding pressure, may indicate the person has shifted their weight and changed position whilst sat down.

The device may be adapted to provide a pressure map, or to monitor pressure at multiple positions, for example. If so, then each monitored position may be used to consider a change in overall pressure distribution by comparing first and second pressure distributions. This may be done by using a suitable statistical method to assess whether there has been enough of a change in pressure applied at each point (possibly by assigning a suitable weighting to each point) to consider that the person's seated position has significantly changed relative to whatever starting pressure distribution is used. Whether a change in seated position is significant is considered with respect to whether pressure sore risk is substantially reduced by the change.

The comparison may take into account or compensate for gradual compression of a foam cushion over time, for example.

The device may be configured to differentiate data recorded in respect of a designated device user and data recorded in respect of another device user or users. The device may be configured to record weight and/or pressure distribution data, for example using a load sensor and/or pressure mapping means. The device may be configured to detect or recognise a tag or user device (such as a mobile phone or electronic tag) in proximity to the device for corroborating user identity when sat on or over the device.

This can be used to distinguish between data for the intended device user and data recorded for other users. For example, in a care home setting, it is possible that different people (whether resident or visiting) may sit in a chair which has one person's pressure monitoring device. If the device is customised to that particular person, for example because it is incorporated into a support cushion which provides a custom degree of support, this can add noise or erroneous data points associated with the designated user and may adversely affect interpretation of the data and subsequent actions taken in respect of that designated user.

The data may be tagged or marked to highlight that it does not appear to correspond to a designated user. The device may be configured to store data in a separate database if it appears to correspond to a user that is not the designated user.

The pressure monitoring device may be disposed in a body of a mat for placement on or under or in a body of a cushion, mattress or structure on which the person sits or reclines during use. The mat may also be placed inside a pocket of a cushion, mattress or structure on which the person sites or reclines during use.

The device may be in the form of a flexible cover for fitting over a cushion, mattress or structure on which the person sits or reclines during use.

This allows for a conventional cushion or mattress to be retrofitted with the device, keeping costs down for institutions such as care homes that may purchase the devices.

The flexible cover may be made of an extensible or stretchy material.

The flexible cover may be provided in a size specific to a particular cushion or seat.

The flexible cover may include one or more pockets for receiving any one or more of: the pressure sensing means (including one or more of the pressure switches), the data logger, and the communication means.

The flexible cover may include a receiving space for the cushion, mattress or structure on which the person sits or reclines during use.

The flexible cover may include engagement means such as an elasticated (or partially-elasticated) periphery or drawstring(s) for assisting the flexible cover to remain in engagement with the cushion, mattress or structure. The engagement means may be releasable engagement means. Other engagement means or closures such as poppers, zips or other co-operating closures may be provided to achieve the same thing.

The flexible cover may be a fabric cover. The fabric cover may include a breathable material.

The flexible cover may be suitable for machine-washing. Some or all of the pressure sensing means, data logger and communication means may be removable from the cover to facilitate this.

The flexible cover may be considered as (part of) a kit for retrofitting to a conventional cushion, mattress or other seat. That is, a kit for converting or upgrading a conventional cushion, mattress or other seat into a pressure sensing cushion, pressure sensing mattress or pressure sensing seat.

According to a second aspect of the present invention there is provided a system for clinical use in monitoring activity of a person on a cushion, mattress, or other structure supporting the person in a seated or reclined position. The system comprises a pressure monitoring device (preferably a device according to the first aspect of the invention) and an external device.

The pressure monitoring device comprises:
a pressure sensing means for sensing pressure exerted by the person, the pressure sensing means being configured to sense: pressure at or below a first pressure threshold for corresponding to the absence of the person, pressure above the first pressure threshold and at or below a second pressure threshold for corresponding to the seated or reclined person generating acceptable pressure, and pressure above the second pressure threshold for corresponding to the seated or reclined person generating excessive pressure;
a data logger connected to the pressure sensing means and configured to monitor the pressure sensing means, in which the data logger is configured to record pressure data over a continuous period of time each time the pressure applied to the cushion drops below or exceeds the first pressure threshold and each time the pressure applied to the cushion drops below or exceeds the second pressure threshold; and
a communication means connected to the data logger for selectively establishing a connection to an external electronic device and transmitting recorded data.

The external device comprises a communication means for receiving data from the pressure monitoring device, and a storage means for storing the received data. The external device selectively establishes a connection to the pressure monitoring device and the data stored in the data logger is transmitted to the external device.

The second aspect of the invention has at least all the same advantages as the first aspect of the invention. The second aspect of the invention may include any features or combination of features presented with respect to the first aspect of the invention.

The system may include a remote server. The external device may selectively establish a connection to the remote server. The external device may transmit the pressure data to the remote server through its communication means. The remote server may store the pressure data received from the external device.

The remote server may process the received pressure data. The server may interpret the pressure data and/or analyse the pressure data. The remote server may generate pressure information based on the processed pressure data. The pressure information may be transmitted to the external device. The pressure information may be a subset of the pressure data, and/or a visual form of the pressure data.

A graphical output may be displayed on the external device. The graphical output may be a graph that represents the use of the seat, cushion, mattress, or structure over a period of time, such as the use of a cushion over a day or part of the day. The graph may show the pressure data or pressure information over time, for example over a day or part of a day. For example, the graph may show the pressure data or pressure information over a 24-hour period, a 2-hour period, a 4-hour period, a 12-hour period, an 18-hour period or any other suitable period. The graphical display allows the user of the external device to review the usage of the seat, cushion, mattress or other structure.

The external device may transmit control parameters, instructions and/or data to the pressure monitoring device. The control parameters may include the set time period for the timer, the pressure thresholds and/or any other parameters governing the control of the pressure monitoring device.

The system may comprise a plurality of pressure monitoring devices, the external device may selectively establish a connection with one of the pressure monitoring devices.

The pressure monitoring device in the second aspect of the invention may be the pressure monitoring cushion of the first aspect of the invention.

The system may be configured to determine a change of position of the person on the pressure monitoring device. The external device may be configured to produce a change of position notification.

The change in position may be determined based on the pressure data over a time period. The pressure monitoring device may be configured to determine whether there has been a change of position. The pressure monitoring device may be configured to record one or more instances of the change of position for providing information on the person's change in position on the seat to the external electronic device. The pressure monitoring device may be configured to transmit one or more instances of the change of position.

The external device may determine whether there has been a change of position.

The server may be configured to determine whether there has been a change of position.

According to a third aspect of the present invention there is provided a method of managing pressure sore risk by monitoring activity of a person using a seat such as a cushion, mattress or other structure supporting the person in a seated or reclined position, the method comprising:
connecting, by wireless communication means, an external device to a pressure monitoring device, the pressure monitoring device being disposed on, in or under the seat, and recording pressure data from a pressure sensing means for sensing pressure exerted by the person;
determining, using the pressure data for a time period since the person began using the seat or since a previous check for change of position was carried out, whether the person seated or reclined on the seat has substantially changed their seated or reclined position within the time period by providing pressure data or information about the same to the external device (such as displaying a graph of the pressure data), or providing to the external device a notification related to whether the person has substantially changed their seated or reclined position, for use in making a clinical decision or assessment in relation to that person, such as instructing the person to change position.

This enables a nurse, doctor or care worker (or another person involved in caring for a person / patient who has or is at risk of developing pressure sores) to quickly check whether the person / patient has changed their seated position, compared to the time they first sat down or in the time since the check was previously carried out.

This is useful because the person may not accurately remember whether they have adjusted their seated position over a particular time period, or whether they got up and sat back down in a different position. A nurse or care worker cannot monitor a patient perpetually- they have a multitude of other things to do, and generally need to care for a plurality of patients at any one time. Thus, having a reliable, objective means of determining whether a given patient has actually substantially changed position whilst the carer is not present is useful to inform an assessment of clinical risk of pressure sores and whether any action is needed at a given time.

For example, a care worker may visit a patient and quickly confirm, via the external device, whether there is any indication that that particular patient has changed seated position or gotten up in the two hours (or such period as may apply) since they last checked in on that person. If the person / patient has altered their seated position in a substantial way, then the pressure sore risk is mitigated, at least temporarily. If the person has not changed position, then the carer can take positive action to remind them or help them to change position. This can also avoid a scenario where multiple care workers each assume or believe another care worker has attended to a particular patient.

Similarly, a notification from the external device may prompt the carer to visit a particular patient in the event that a change of position has not occurred in a particular timeframe.

A smartphone or tablet, or another external device, may be used to initiate an on-demand check for a change of a person's seated position as each person is visited by a carer. The wireless connection from monitoring device to the smartphone (or similar) may function over at least 1m or 2m distance, so that the check can be performed in a socially-distanced manner (if circumstances at the time so require). Alternatively, the smartphone or similar device may be sent data automatically or periodically by the pressure monitoring device, such as by either a direct connection (e.g. Bluetooth (RTM)) or via Wi-Fi (RTM), or by NFC or infrared connection, for example. For an NFC connection, a device may need to be within a few centimetres of the device, or substantially in contact with the device.

According to another aspect of the invention, there is provided a pressure monitoring device as set out in claim 30.

Any feature or features in presented with respect to one aspect of the invention may be independently provided in any of the other aspects of the invention.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows a top perspective view of a pressure monitoring cushion incorporating a pressure monitoring device according to the present invention, with an external covering removed;
Figure 2 shows an underside perspective view of the pressure monitoring cushion in Figure 1;
Figure 3 shows an enlarged perspective view of a corner of the pressure monitoring cushion in Figure 2;
Figure 4 shows a schematic representation of a system for clinical use in monitoring activity of a person using a pressure monitoring cushion incorporating a pressure monitoring device according to the present invention;
Figure 5 shows an example of a graph displaying data collected from a pressure monitoring cushion incorporating a pressure monitoring device according to the present invention;
Figure 6 shows a perspective view of a pressure monitoring device in the form of a mat for use with a cushion, according to a second embodiment of the invention;
Figure 7 shows a first perspective view of a flexible cover for covering a cushion, in which the flexible cover contains the pressure monitoring device of Figure 6; and
Figure 8 shows a second perspective view of the flexible cover of Figure 7, fitted to a cushion.

### DESCRIPTION OF PREFERRED EMBODIMENTS

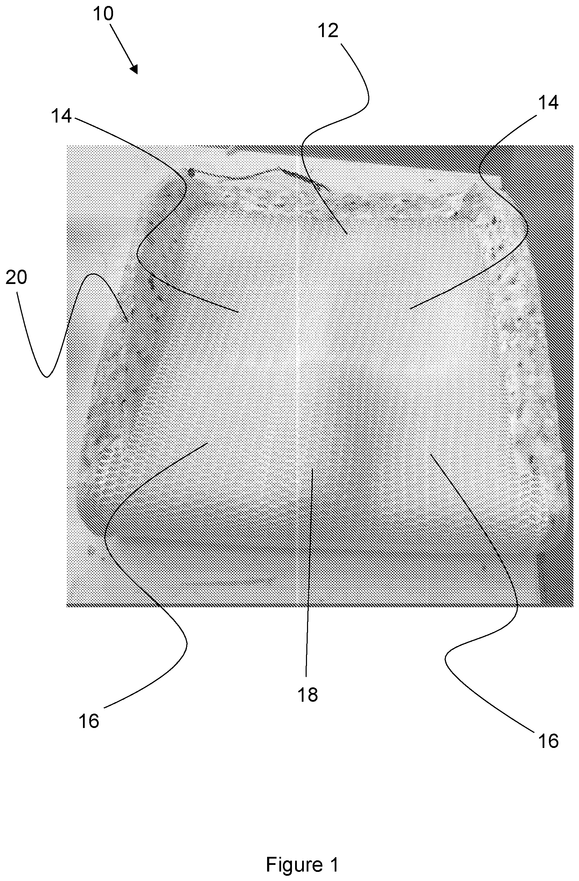

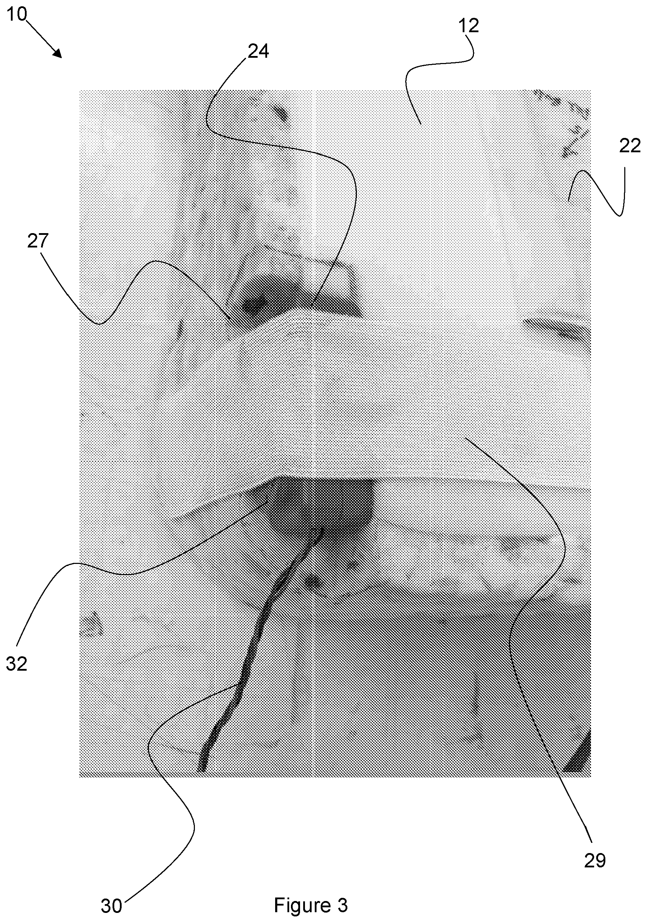

Referring to Figures 1, 2, 3 and 4 a pressure monitoring cushion incorporating a pressure monitoring device is generally indicated at 10. It will be appreciated that the cushion 10 is shown without an external covering, but that the cushion is intended to include a fabric covering prior to use by a person. The cushion 10 in this embodiment is a discrete item for placement on an existing seat or bed (that is, on top of a seat squab or mattress which may already be cushioned), but embodiments are also contemplated where the cushion may be part of a seat or bed, for example.

The cushion includes a cushion body 12. The cushion body 12 is substantially cuboidal, having an approximately square footprint. In this embodiment, the cushion body 12 is made from a resilient foam which can be compressed. However, it will be appreciated that any suitable support medium may be provided for the cushion body. A mesh (here a hexagonal mesh) extends over the cushion body 12 for wicking moisture away.

An upper surface of the cushion body is contoured for a portion of the human body. Two recessed portions 14 for receiving a person's buttocks are provided in the contoured upper surface proximate the rear of the cushion body 12. Two leg receiving portions 16 are also provided in the contoured upper surface proximate the front of the cushion body 12. A protrusion 18 is provided between the two leg receiving portions. The protrusion runs longitudinally between the leg receiving portions 16.

A flange 20 with a tapered wall is provided on either side of the cushion body extending from the upper surface. The flange 20 also includes a portion at the back of the cushion 10, although it is of reduced height relative to the side portions in this embodiment. The flange 20 with tapered wall creates a bucketed seating surface which provides support to a user. In some embodiments, a flange may be provided along the rear side of the cushion body and join the flanges on the sides of the cushion.

The components of the pressure monitoring device will discussed below.

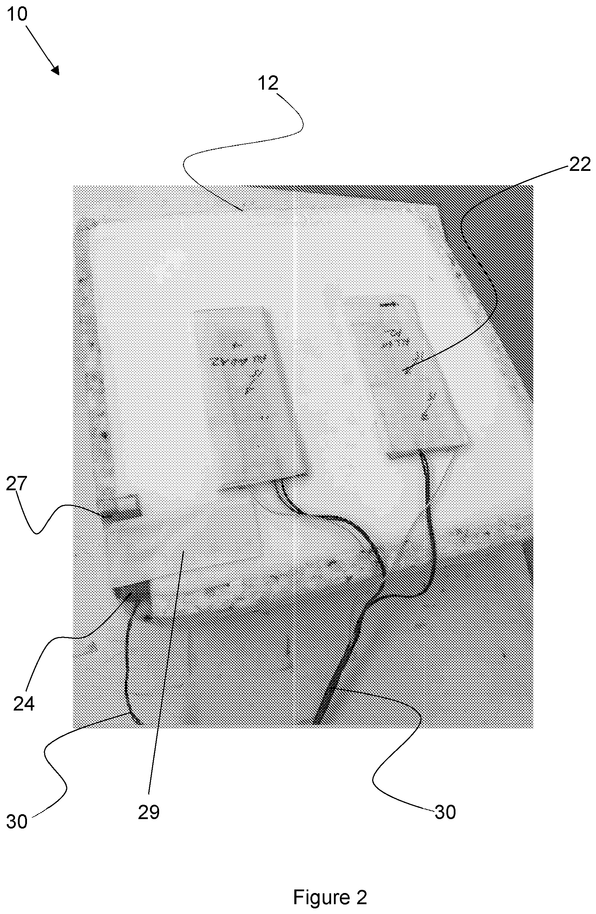

As shown in Figure 2, two pressure sensor units 22 are disposed on or in a lower surface, or base, of the cushion body 12. These sensor units provide the pressure sensing means. In the current embodiment, the pressure sensor units comprise pressure switches. In other embodiments, the pressure sensor units may measure a pressure value in an analog sense.

The pressure switches are configured to sense whether pressure exceeds a set of predetermined threshold values. A first pressure threshold is set which corresponds to the absence of the person on the cushion or negligible weight (relative to that of a person). Pressure above that first threshold but below a set second pressure threshold corresponds to the person sat or reclined on the cushion at acceptable pressure, typically without being at a significantly increased risk of pressure sore formation. Pressure above the second pressure threshold corresponds to the person sat or reclined on the cushion where excessive pressure is being generated.

It will be appreciated that the exact pressure threshold values will vary depending on the person involved, because each person has different clinical needs. It will also be appreciated that one or more additional pressure thresholds may be set which are within or beyond the above ranges.

In some embodiments, for example, a first pressure threshold may be set at around 10665.8 Pa (80mmHg).

In some embodiments, for example, a second pressure threshold may be set at around 18665,1 Pa (140mmHg). The invention is defined by the appended claims.

The pressure sensor units 22 are disposed underneath the area typically occupied by the user's pelvis, i.e. a pressure sensor unit is disposed underneath a recess 14. In some embodiments, an array of sensors may be provided.

The pressure sensor units 22 may be substantially proud, substantially flush or substantially shy of the lower surface, or base, of the cushion body 12. The pressure sensor units 22 may be secured to the cushion by an adhesive, or other retention means such as a strap or hook and loop type fastener.

A housing 24 is provided to enclose a data logger 25, communication means 26 and other electronic or electrical components. The housing 24 is provided in a recess 27 formed in a rear corner of the lower surface of the cushion body 12. The recess 27 is sized and shaped so that the housing 24 is seated within the confines of the cushion body 12, i.e. the housing 24 is substantially flush or substantially shy of the lower surface and side surfaces of cushion body 12.

A battery may be provided in the housing 24.

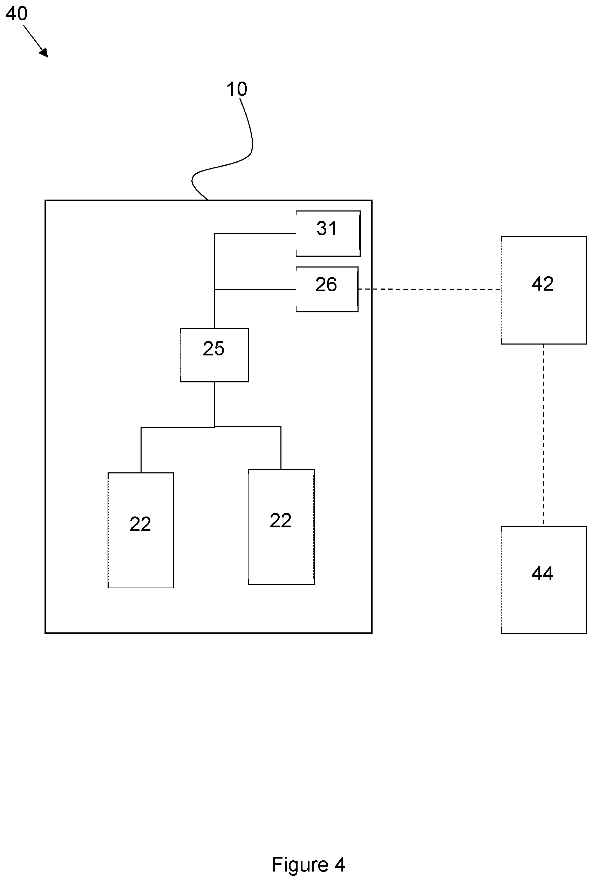

The data logger 25, communication means 26 and other electrical and electronic components inside the housing 24 are discussed in more detail below and shown in Figure 4.

The data logger 25 is connected to the pressure sensor units 22 and is configured to record pressure data over a period of time. In particular, each time the pressure applied to the cushion drops below or exceeds any of the pressure thresholds, the data logger records relevant pressure data. The data logger may also store time and/or date data.

The data logger may be provided in a control unit, or act as the control unit, for controlling functions of the pressure monitoring cushion 10.

The pressure threshold may represent a cushion use state. A non-use state is based on the first pressure threshold which can be set to represent zero or a small pressure, preferably the first pressure threshold should be non-zero and lower than the typical weight of an average, or specific, patient using the cushion. An acceptable use state is based on the second pressure threshold which could be set to represent the pressure usually exerted by an average, or specific, patient using the cushion, or a recommended pressure.

A further use state may be based on a third pressure threshold, or simply a pressure greater than the second pressure threshold. The third pressure threshold is higher than both the first and second pressure thresholds. The third threshold will typically be a higher pressure than usually exerted by an average, or specific, patient using the cushion, or the recommended pressure.

The cushion or device can identify a change of seated position of the person sat on the cushion. For example, a baseline or average of the pressure exerted in one or more areas may be determined from the pressure data. Changes in pressure relative to the baseline or average may be used to identify that the person has changed their seated position. In another example, a change in state which is apparent in the data or a graphical display thereof may be used to identify that or assess whether a person has changed their seated position. Once identified, this may be recorded in the memory of the data logger and/or transmitted to an external device. In some embodiments, the pressure monitoring device or the external device may identify and/or record the change of position.

A timer may be provided. The timer may be configured to run for a set time period beginning when pressure data from the cushion indicates that a person has just sat down or reclined on the cushion. The set time period may be set by the user of an external device that has established a connection with the cushion 10. The set time period may also dynamically change based on the pressure data. If the duration for which the person is sat down exceeds the set time period, then a warning or notification may be sent to the external device.

The two-way communication means 26 is preferably a wireless communication means, and more preferably a short-range wireless communication means. The communication means provides a means to transmit data stored in the data logger 25 and receive data or instructions.

A retaining strap 29 is provided to retain the housing 24 in the recess 27. The retaining strap 29 extends from the lower surface to one side of the cushion. The retaining strap may include an elastic material so as to retain the housing 24 within the recess 27. Each end of the retaining strap 29 is secured to the cushion body by an adhesive or other means such as hook and loop type fasteners.

A wiring loom 30 is provided to connect each pressure sensor unit 22 to the data logger and electronic or electrical components within the housing 24. The wiring loom 30 includes a plurality of wires which are used to transmit signals and power between the pressure sensor units 22 and the data logger and electronic or electrical components. The wiring loom 30, or plurality of wires, pass through an opening in the housing 24.

A sounder 31 may be provided in the housing 24. A plurality of apertures 32 may be provided in the housing 24 to allow transmission of the sound wave produced by the sounder. The apertures 31 may also provide a means to allow air to flow between inside and outside the housing 24.

A cover (not shown), preferably a removable cover, may be provided to cover the pressure monitoring cushion 10. The cover may be made from a material which is suitable for cleaning, it may also be waterproof. The cover acts as a boundary between the patient and the cushion body 12.

In use, a patient, or other user, sits or is lowered onto the upper surface of the pressure monitoring cushion 10 so that their pelvis is disposed in the recesses 14 and their thighs are received in the thigh receiving regions 16. The seated or reclined patient exerts a pressure downwards. The exerted pressure is sensed by the pressure sensor units 22. The data logger 25 receives pressure data from the sensor units 22 and stores it in a memory. The pressure data stored in the data logger's memory is retrievable by an external device once connected.

If the person gets up or changes their seated position enough to exceed the pressure threshold(s), the data logger 25 records pressure data for the event. This may repeat until the cushion's battery power runs out.

Referring to Figure 4, a system for monitoring use of a pressure monitoring cushion incorporating a pressure monitoring device is generally indicated at 40.

In the current embodiment, the pressure monitoring cushion 10 provided is the same as discussed above and as such reference numerals will be maintained.

An external device 42, such as a computer, smart device, mobile phone, or tablet, is provided. The external device 42 may be used by a carer, clinician, therapist or patient. The external device can selectively establish communication with the pressure monitoring cushion 10 through a two-way communication means, preferably a wireless communication means.

The external device 42 selectively connects to the pressure monitoring cushion 10 through the communication means. Once connected, data stored in the data logger 25 is transmitted to the external device 42 and stored in a memory of the external device.

The external device 42 may also transmit data and instructions to the pressure monitoring cushion 10. For example, the external device may be used to set parameters such as the set time period of the timer or pressure thresholds etc.

A remote server 44 may also be provided. The external device 42 may establish a connection with the remote server 44. The pressure data stored in the external device may be the transmitted to the remote server for analysis. The pressure data may be stored in a database in the remote server 44. This data may be stored so as to be uniquely identifiable with a particular pressure monitoring cushion.

The remote server 44, and in some embodiments the external device 42, may process the data to determine pressure information, such as the usage of the cushion of a period of time. In some embodiments, where the actual value of pressure is measured, this could include categorising the pressure measurements over that time into at least three categories. The categories may include 'optimal', 'monitor' and 'attention' as pressure sore categories of increasing risk for a clinician to consider, for example.

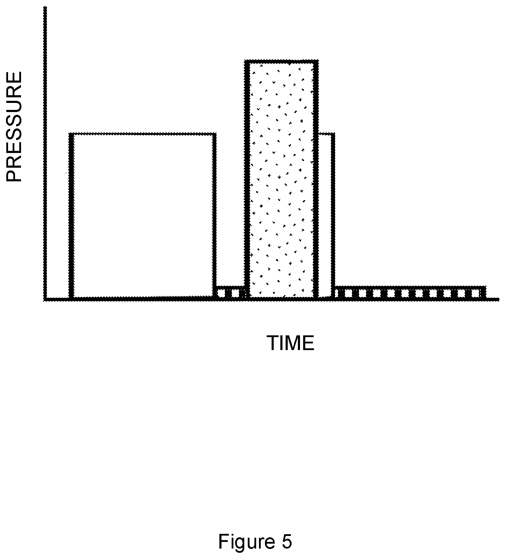

The external device may selectively access the pressure data stored on the remote server 44 to show on a display the pressure information or pressure data over a particular period of time. The display may also display the pressure data in a manner useful to the carer or patient, i.e. the display may show a graph sets out the pressure for that time period. For example, a graph such as that shown in Figure 5 may be displayed on the external device.

Figure 5 shows cushion usage over a 24 hour period plotted against pressure. The graph does not display the actual pressures measured in those periods. The pressure data has been categorised, by the server, into one of three categories, optimal (shown as the striped bars with the smallest pressure), monitor (show as the empty bars with a middling pressure) and attention (shown as the stippled bar with the highest pressure). This categorisation allows the carer or user to easily identify periods which may be of concern.

In some embodiments of the system, such as that used on a ward or in a care home, a plurality of pressure monitoring cushions may be provided. The external device 42 may establish a connection to a user selected cushion 10, i.e. the user of the external device 42 may select which cushion out of the plurality of cushion to connected to. Once connected, the external device may control the cushion's indicator so that they can validate which is the connected cushion.

The invention is defined by the appended claims.

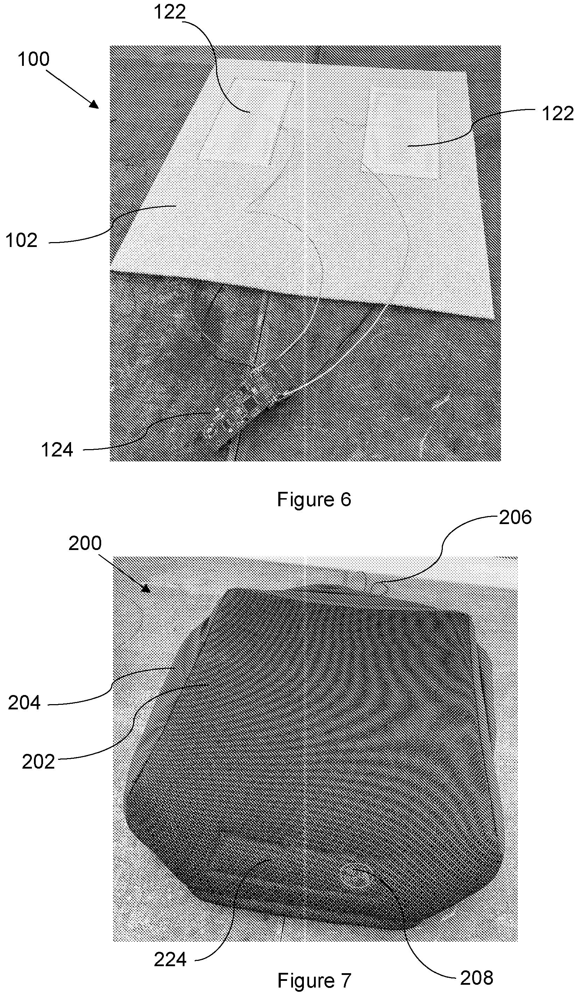

Figure 6 shows a second embodiment of a pressure monitoring device, indicated generally at 100. The device 100 functions and can be used in a similar way to that of Figures 1-5, particularly when placed on, in or under a cushion.

The device includes a mat 102 which in this embodiment is approximately square. The mat 102 in this embodiment is made of a fabric such as a felt, but any suitable material may be used. The material is preferably flexible.

A pair of sensor units or packs 122 are provided in left and right halves of the mat 102. The sensor units 122 can be the same or similar to the units 22 described above, including how they function. In some embodiments, the units 122 may include one or more pairs of flexible conductors, with neighbouring conductors each separated by an apertured electrical insulator. The thickness of the electrical insulator can affect the magnitude of the pressure switch threshold.

The sensor units 122 are connected by wires to a PCB 124 which includes the data logger and communication means, and any preferred electronic components such as a timer, a sounder and/or other components from the housing 24 of the first embodiment described earlier. The data logger is programmed similarly to the first embodiment, and the communication means can be the same too. In preferred embodiments, the communication means is NFC-enabled.

The PCB 124 may be provided in a protective housing in some embodiments, which may be a rigid housing. The wires include wires for detecting whether the pressure sensors in the first and second units 122 have been triggered, and if so to which pressure threshold (in this case, above a first threshold or above a second threshold).

Figure 7 shows a flexible cover, indicated at 200, which includes the device 100 discussed with respect to Figure 6. The cover 200 is suitable for fitting over a cushion, but is empty in Figure 7, i.e. is not fitted to a cushion.

The cover 200 includes a top surface 202, which in this embodiment is a mesh made of a breathable material such as cotton or nylon. It will be appreciated that a user-specific identifier or surface indicia may be provided on the top surface. This may include any one or more of: a particular colour or combination or colours, a pattern or patterns, a number, one or more letters or words (such as a name), an image such as a face, or any other unique identifier that can be easily seen and interpreted by human vision to indicate a particular person.

A skirt or flexible sidewall 204 is provided around the top surface 202, particularly around the left side, rear and right side. The top surface 202 may form a recess at the front for seating or aligning a cushion in the cover 200. The skirt 204 may in some embodiments be provided at the front as well. The skirt 204 is made of a breathable material in this embodiment.

A drawstring 206 is connected to the skirt 204 for drawing the skirt tightly around a cushion. The drawstring 206 is provided at the rear in this embodiment, but may be provided at any other suitable position. It will be appreciated that other engagement means may be provided in other embodiments, such as an elasticated edge.

The top surface 202 and skirt 204 define a receiving space for fitting a cushion. The receiving space is below the top surface 202 in the orientation of the cover 200 shown in Figure 7.

Indicia 208 indicating a wireless communication region (in this case, NFC) is disposed on the top surface 202. In this case, it is located towards the front of the cover 200.

A pocket 224 containing the PCB 124 is provided at the front of the cover 200 in this embodiment. The pocket may be a rigid or protective pocket to prevent the PCB being damaged. The indicia 208 are provided on the front of the pocket 224 in this embodiment, although it will be appreciated that this is not essential.

The top surface 202 can include a pocket on its underside for receiving the mat 102 in some embodiments. In other embodiments, the mat 102 may be secured to the top surface by fixing means, such as adhesive or connectors.

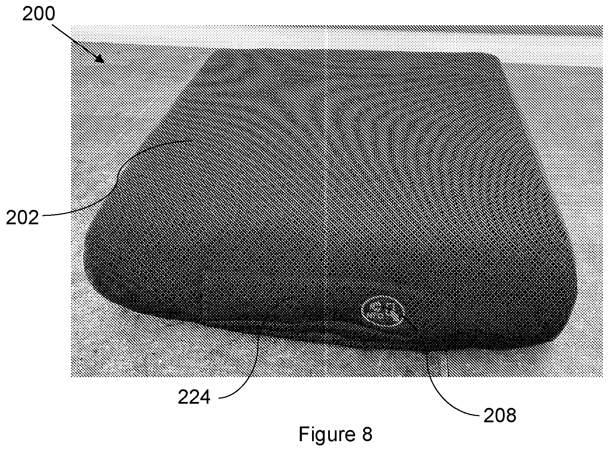

Figure 8 shows the cover 200 fitted to a cushion. That is, the cushion is disposed in the cushion receiving space of the cover. The top surface 202 and skirt 204 are stretched substantially taut. The drawstring 206 is pulled tight and tied off or secured against a loss of tension for maintaining the cover 200 on the cushion.

The sensor packs 122 are located directly underneath the top surface 202 of the cover 200, spaced apart on top of the cushion. The PCB 124 is located at the front side of the cushion, although it will be appreciated that it may be provided on another side of the cushion in other embodiments.

It will be appreciated that the cover may be provided in any suitable size for fitting to any cushion, mattress or other seat squab/structure on which a person may sit or recline.

It will also be appreciated that in any of the above embodiments, the data logger (or other microchip or processor) may be programmed to identify data recorded in respect of a first (designated) device user and to identify data which is suspected of relating to device use by a one or more other device users. This may be done by sensing any one or more of total weight, pressure distribution or a person-specific tag (for example the presence or absence of a particular electronic device) and using that to assess whether each instance of data relates to the same person, although other means of achieving the same are also contemplated.

In other words, the data logger may be able to differentiate between different users, and so differentiate the recorded data accordingly by marking (or not marking) or storing (or not storing) the data in a way that identifies it as relating to a non-designated user for that pressure monitoring device.

## Claims

1. A pressure monitoring device (10) selected from a cushion, a mat, and a cushion cover for clinical use in monitoring the activity of a person on a seat, mattress or other structure for supporting the person in a seated or reclined position, the device (10) comprising:
a pressure sensing means for sensing pressure exerted by the person, includes a first pressure switch configured to operate at a first pressure threshold and a second pressure switch configured to operate at a second pressure threshold, the pressure sensing means being configured to sense: pressure at or below the first pressure threshold for corresponding to the absence of the person, pressure above the first pressure threshold and at or below the second pressure threshold for corresponding to the seated or reclined person generating acceptable pressure, and pressure above the second pressure threshold for corresponding to the seated or reclined person generating excessive pressure;
a data logger (25) connected to the pressure sensing means and configured to monitor the pressure sensing means, in which the data logger (25) is configured to record pressure data over a continuous period of time which includes recording each time the pressure applied to the cushion drops below or exceeds the first pressure threshold and each time the pressure applied to the cushion drops below or exceeds the second pressure threshold; and
a communication means (26) connected to the data logger (25) for selectively establishing a connection to an external electronic device (42) and transmitting recorded data.

2. A pressure monitoring device (10) as claimed in claim 1, in which the pressure sensing means includes first and second pressure sensor units (22) horizontally spaced apart for occupying different positions underneath a seated or reclined person.

3. A pressure monitoring device (10) as claimed in claim 1 or claim 2, in which the pressure sensing means includes at least four pressure sensors for monitoring pressure in four respective quadrants underneath the user.

4. A pressure monitoring device (10) as claimed in any preceding claim, in which the communication means (26) is a two-way communication means and/or a wireless communication means, optionally a short-range wireless communication means or NFC.

5. A pressure monitoring device (10) as claimed in any preceding claim, in which an alert means is provided for alerting the seated or reclined person to shift their position, and the alert means includes a timer which is in use configured to run for a set time period beginning when pressure data indicates that a person has just sat down or reclined on the cushion, mattress or other structure, and the alert means being configured to initiate when the time period has elapsed.

6. A pressure monitoring device (10) as claimed in claim 5, in which the set time period is determined or altered based on the sensed pressure and optionally in which the set time period is during use reduced based on the sensed pressure exceeding the second pressure threshold.

7. A pressure monitoring device (10) as claimed in any preceding claim, further comprising an indicator or user-specific surface indicia for providing a way of identifying the device (10) from a plurality of substantially similar devices, optionally in which the indicator includes a sounder (31) controllable by the external device (42) through the communication means (26).

8. A pressure monitoring device (10) as claimed in any preceding claim, in which the device (10) is configured to identify changes in the pressure data which correspond to a change of position of the person during use, and to record and/or transmit one or more instances of the change of position for providing information on the person's change in position on the seat to the external electronic device (42).

9. A system (40) for clinical use in monitoring activity of a person on a cushion, mattress, or other structure supporting the person in a seated or reclined position, the system (40) comprising:
a pressure monitoring device (10) as claimed in any preceding claim; and
an external electronic device (42) comprising a communication means (26) for receiving pressure data from the pressure monitoring cushion, and a data storage means for storing the pressure data,
wherein in use the external device (42) establishes a connection to the pressure monitoring device (10) and the data stored in the data logger (25) is transmitted to the external electronic device (42).

10. A system (40) as claimed in claim 9, further comprising a server (44), the external device (42) in use establishing a connection to the server (44) and transmitting the pressure data to the server (44) through the communication means (26) for storage.

11. A system (40) as claimed in claim 10, in which in use the server (44) processes the received pressure data to produce pressure information and optionally the server (44) transmits the pressure information to the external device (42).

12. A system (40) as claimed in any of claims 9 to 11, in which a graphical output of the pressure data is displayed on the external device (42), the graphical output including a graph showing pressure switch states to indicate the use of the cushion, mattress, or structure over a period of time.

13. A system (40) as claimed in any of claims 9 to 12, in which a plurality of pressure monitoring devices (10) are provided, and in use the external device (42) establishes a connection to one or more of the plurality of pressure monitoring devices (10).

14. A system (40) as claimed in any of claims 9 to 13, in which the system (40) is configured to determine a change of position of the person on the pressure monitoring device (10), and the external device (42) is configured to produce a change of position notification, optionally in which the change in position is determined based on the pressure data over a time period, wherein:
the pressure monitoring device (10) is configured to determine whether there has been a change of position, and to record and/or transmit one or more instances of the change of position for providing information on the person's change in position on the seat to the external electronic device (42); or
the external device (42) or server (44) is configured to determine whether there has been a change of position.

15. A method of managing pressure sore risk by monitoring activity of a person using a seat, mattress or other structure supporting the person in a seated or reclined position, the method comprising:
connecting, by wireless communication means (26), an external device (42) to a pressure monitoring device (10) as claimed in any of claims 1 to 8, the pressure monitoring device (10) being disposed on, in or under the seat for recording pressure data using a pressure sensing means configured to sense pressure exerted by the person; and
determining, using pressure data for a time period since the person began using the seat or since a previous check for change of position was carried out, whether the person seated or reclined on the seat has substantially changed their seated or reclined position within the time period by providing information at the external device (42), or providing a notification on the external device (42), related to the pressure data for use in making a clinical decision or assessment in relation to whether that person has substantially changed their seated or reclined position, such as whether to instruct the person to change position.

## Patentansprüche

1. Drucküberwachungsvorrichtung (10), ausgewählt aus einem Polster, einer Matte und einem Polsterbezug für die klinische Verwendung zur Überwachung der Aktivität einer Person auf einem Sitz, einer Matratze oder einer anderen Struktur zum Stützen der Person in einer sitzenden oder liegenden Position, wobei die Vorrichtung (10) umfasst:
ein Druckerfassungsmittel zum Erfassen von Druck, der von der Person ausgeübt wird, einschließlich eines ersten Druckschalters, der so konfiguriert ist, dass er bei einer ersten Druckschwelle arbeitet, und eines zweiten Druckschalters, der so konfiguriert ist, dass er bei einer zweiten Druckschwelle arbeitet, wobei das Druckerfassungsmittel so konfiguriert ist, dass es Folgendes erfasst: Druck bei oder unterhalb der ersten Druckschwelle, der der Abwesenheit der Person entspricht, Druck oberhalb der ersten Druckschwelle und bei oder unterhalb der zweiten Druckschwelle, der der sitzenden oder liegenden Person entspricht, die einen akzeptablen Druck erzeugt, und Druck oberhalb der zweiten Druckschwelle, der der sitzenden oder liegenden Person entspricht, die einen übermäßigen Druck erzeugt;
einen Datenlogger (25), der mit dem Druckerfassungsmittel verbunden und so konfiguriert ist, dass er das Druckerfassungsmittel überwacht, wobei der Datenlogger (25) so konfiguriert ist, dass er Druckdaten über einen kontinuierlichen Zeitraum aufzeichnet, was die Aufzeichnung jedes Mal einschließt, wenn der auf das Polster ausgeübte Druck die erste Druckschwelle unterschreitet oder überschreitet, und jedes Mal, wenn der auf das Polster ausgeübte Druck die zweite Druckschwelle unterschreitet oder überschreitet; und
ein Kommunikationsmittel (26), das mit dem Datenlogger (25) verbunden ist, um selektiv eine Verbindung zu einer externen elektronischen Vorrichtung (42) herzustellen und aufgezeichnete Daten zu übertragen.

2. Drucküberwachungsvorrichtung (10) nach Anspruch 1, wobei das Druckerfassungsmittel erste und zweite Drucksensoreinheiten (22) einschließt, die horizontal voneinander getrennt sind, um verschiedene Positionen unter einer sitzenden oder liegenden Person einzunehmen.

3. Drucküberwachungsvorrichtung (10) nach Anspruch 1 oder Anspruch 2, wobei das Druckerfassungsmittel mindestens vier Drucksensoren zum Überwachen des Drucks in vier jeweiligen Quadranten unterhalb des Benutzers einschließt.

4. Drucküberwachungsvorrichtung (10) nach einem der vorhergehenden Ansprüche, wobei das Kommunikationsmittel (26) ein Zwei-Wege-Kommunikationsmittel und/oder ein drahtloses Kommunikationsmittel, optional ein drahtloses Kommunikationsmittel mit kurzer Reichweite oder NFC, ist.

5. Drucküberwachungsvorrichtung (10) nach einem der vorhergehenden Ansprüche, wobei ein Alarmmittel vorgesehen ist, um die sitzende oder liegende Person zu warnen, ihre Position zu verlagern, und das Alarmmittel einen Timer einschließt, der im Gebrauch so konfiguriert ist, dass er für einen festgelegten Zeitraum läuft, der beginnt, wenn Druckdaten anzeigen, dass sich eine Person gerade auf das Polster, die Matratze oder eine andere Struktur gesetzt oder gelegt hat, und wobei das Alarmmittel so konfiguriert ist, dass es ausgelöst wird, wenn der Zeitraum abgelaufen ist.

6. Drucküberwachungsvorrichtung (10) nach Anspruch 5, wobei der festgelegte Zeitraum basierend auf dem erfassten Druck bestimmt oder verändert wird und wobei optional der festgelegte Zeitraum während der Verwendung basierend auf dem erfassten Druck, der die zweite Druckschwelle überschreitet, reduziert wird.

7. Drucküberwachungsvorrichtung (10) nach einem der vorhergehenden Ansprüche, ferner umfassend eine Anzeige oder benutzerspezifische Oberflächenmarkierungen zum Bereitstellen einer Möglichkeit zum Identifizieren der Vorrichtung (10) aus einer Vielzahl von im Wesentlichen ähnlichen Vorrichtungen, wobei optional die Anzeige einen durch die externe Vorrichtung (42) über das Kommunikationsmittel (26) steuerbaren Schallgeber (31) umfasst.

8. Drucküberwachungsvorrichtung (10) nach einem der vorhergehenden Ansprüche, wobei die Vorrichtung (10) so konfiguriert ist, dass sie Änderungen der Druckdaten identifiziert, die einer Positionsänderung der Person während der Verwendung entsprechen, und eine oder mehrere Instanzen der Positionsänderung aufzeichnet und/oder überträgt, um Informationen über die Positionsänderung der Person auf dem Sitz an die externe elektronische Vorrichtung (42) bereitzustellen.

9. System (40) zur klinischen Verwendung zur Überwachung der Aktivität einer Person auf einem Polster, einer Matratze oder einer anderen Struktur, die die Person in einer sitzenden oder liegenden Position stützt, wobei das System (40) umfasst:
eine Drucküberwachungsvorrichtung (10) nach einem der vorhergehenden Ansprüche; und
eine externe elektronische Vorrichtung (42), die ein Kommunikationsmittel (26) zum Empfangen von Druckdaten von dem Drucküberwachungspolster und ein Datenspeichermittel zum Speichern der Druckdaten umfasst,
wobei im Gebrauch die externe Vorrichtung (42) eine Verbindung zur Drucküberwachungsvorrichtung (10) herstellt und die in dem Datenlogger (25) gespeicherten Daten an die externe elektronische Vorrichtung (42) übertragen werden.

10. System (40) nach Anspruch 9, ferner umfassend einen Server (44), wobei die externe Vorrichtung (42) im Gebrauch eine Verbindung zum Server (44) herstellt und die Druckdaten über die Kommunikationsmittel (26) zum Speichern an den Server (44) überträgt.

11. System (40) nach Anspruch 10, wobei der Server (44) im Gebrauch die empfangenen Druckdaten verarbeitet, um Druckinformationen zu erzeugen, und der Server (44) optional die Druckinformationen an die externe Vorrichtung (42) überträgt.

12. System (40) nach einem der Ansprüche 9 bis 11, wobei eine grafische Ausgabe der Druckdaten auf der externen Vorrichtung (42) angezeigt wird, wobei die grafische Ausgabe ein Diagramm einschließt, das die Zustände des Druckschalters anzeigt, um die Verwendung des Polsters, der Matratze oder der Struktur über einen Zeitraum anzuzeigen.

13. System (40) nach einem der Ansprüche 9 bis 12, wobei eine Vielzahl von Drucküberwachungsvorrichtungen (10) vorgesehen sind und die externe Vorrichtung (42) im Gebrauch eine Verbindung zu einer oder mehreren der Vielzahl von Drucküberwachungsvorrichtungen (10) herstellt.

14. System (40) nach einem der Ansprüche 9 bis 13, wobei das System (40) so konfiguriert ist, dass es eine Positionsänderung der Person an der Drucküberwachungsvorrichtung (10) bestimmt, und die externe Vorrichtung (42) so konfiguriert ist, dass sie eine Positionsänderungsmeldung erzeugt, wobei optional die Positionsänderung basierend auf den Druckdaten über einen Zeitraum bestimmt wird, wobei:
die Drucküberwachungsvorrichtung (10) so konfiguriert ist, dass sie bestimmt, ob eine Positionsänderung stattgefunden hat, und eine oder mehrere Instanzen der Positionsänderung aufzeichnet und/oder überträgt, um der externen elektronischen Vorrichtung (42) Informationen über die Positionsänderung der Person auf dem Sitz bereitzustellen; oder
die externe Vorrichtung (42) oder der Server (44) so konfiguriert ist, dass sie bzw. er bestimmt, ob eine Positionsänderung stattgefunden hat.

15. Verfahren zum Management des Risikos von Druckgeschwüren durch Überwachung der Aktivität einer Person unter Verwendung eines Sitzes, einer Matratze oder einer anderen Struktur, die die Person in einer sitzenden oder liegenden Position stützt, wobei das Verfahren umfasst:
Verbinden, durch ein drahtloses Kommunikationsmittel (26), einer externen Vorrichtung (42) mit einer Drucküberwachungsvorrichtung (10) nach einem der Ansprüche 1 bis 8, wobei die Drucküberwachungsvorrichtung (10) auf, in oder unter dem Sitz zum Aufzeichnen von Druckdaten unter Verwendung eines Druckerfassungsmittels angeordnet ist, das so konfiguriert ist, dass es den von der Person ausgeübten Druck erfasst; und
Bestimmen unter Verwendung von Druckdaten für einen Zeitraum seit dem Beginn der Verwendung des Sitzes durch die Person oder seit einer vorherigen Überprüfung auf Positionsänderung, ob die auf dem Sitz sitzende oder liegende Person ihre Sitz- oder Liegeposition innerhalb des Zeitraums wesentlich verändert hat, durch Bereitstellung von Informationen an die externe Vorrichtung (42) oder durch Bereitstellung einer Benachrichtigung auf der externen Vorrichtung (42) in Bezug auf die Druckdaten zur Verwendung bei einer klinischen Entscheidungsfindung oder Beurteilung, ob die Person ihre Sitz- oder Liegeposition wesentlich verändert hat, z. B. ob die Person angewiesen werden soll, ihre Position zu ändern.

## Revendications

1. Dispositif de surveillance de pression (10) choisi parmi un coussin, un tapis et une housse de coussin pour une utilisation clinique dans la surveillance de l'activité d'une personne sur un siège, un matelas ou une autre structure pour soutenir la personne dans une position assise ou allongée, le dispositif (10) comprenant :
un moyen de détection de pression pour détecter une pression exercée par la personne, inclut un premier pressostat configuré pour fonctionner à un premier seuil de pression et un deuxième pressostat configuré pour fonctionner à un deuxième seuil de pression, le moyen de détection de pression étant configuré pour détecter : une pression inférieure ou égale au premier seuil de pression correspondant à l'absence de la personne, une pression supérieure au premier seuil de pression et inférieure ou égale au deuxième seuil de pression correspondant à la personne assise ou allongée générant une pression acceptable, et une pression supérieure au deuxième seuil de pression correspondant à la personne assise ou allongée générant une pression excessive ;
un enregistreur de données (25) connecté au moyen de détection de pression et configuré pour surveiller le moyen de détection de pression, dans lequel l'enregistreur de données (25) est configuré pour enregistrer des données de pression sur une période de temps continue, ce qui inclut l'enregistrement chaque fois que la pression appliquée au coussin devient inférieure ou supérieure au premier seuil de pression et chaque fois que la pression appliquée au coussin devient inférieure ou supérieure au deuxième seuil de pression ; et
un moyen de communication (26) connecté à l'enregistreur de données (25) pour établir sélectivement une connexion à un dispositif électronique externe (42) et transmettre des données enregistrées.

2. Dispositif de surveillance de pression (10) selon la revendication 1, dans lequel le moyen de détection de pression inclut des première et deuxième unités (22) de capteur de pression espacées horizontalement pour occuper différentes positions sous une personne assise ou allongée.

3. Dispositif de surveillance de pression (10) selon la revendication 1 ou la revendication 2, dans lequel le moyen de détection de pression inclut au moins quatre capteurs de pression pour surveiller la pression dans quatre quadrants respectifs sous l'utilisateur.

4. Dispositif de surveillance de pression (10) selon l'une quelconque des revendications précédentes, dans lequel le moyen de communication (26) est un moyen de communication bidirectionnelle et/ou un moyen de communication sans fil, facultativement un moyen de communication sans fil à courte portée ou NFC.

5. Dispositif de surveillance de pression (10) selon l'une quelconque des revendications précédentes, dans lequel un moyen d'alerte est prévu pour alerter la personne assise ou allongée de changer sa position, et le moyen d'alerte inclut une minuterie qui est configurée, en utilisation, pour fonctionner pendant une période de temps définie commençant lorsque des données de pression indiquent qu'une personne vient de s'asseoir ou de s'allonger sur le coussin, le matelas ou une autre structure, et le moyen d'alerte étant configuré pour se déclencher lorsque la période de temps est écoulée.

6. Dispositif de surveillance de pression (10) selon la revendication 5, dans lequel la période de temps définie est déterminée ou modifiée sur la base de la pression détectée et, facultativement, dans lequel la période de temps définie est réduite, pendant l'utilisation, sur la base du fait que la pression détectée dépasse le deuxième seuil de pression.

7. Dispositif de surveillance de pression (10) selon l'une quelconque des revendications précédentes, comprenant en outre un indicateur ou des indices de surface spécifiques à l'utilisateur pour fournir un moyen d'identifier le dispositif (10) parmi une pluralité de dispositifs sensiblement similaires, dans lequel l'indicateur inclut facultativement un avertisseur sonore (31) pouvant être commandé par le dispositif externe (42) par l'intermédiaire du moyen de communication (26).

8. Dispositif de surveillance de pression (10) selon l'une quelconque des revendications précédentes, dans lequel le dispositif (10) est configuré pour identifier des changements des données de pression qui correspondent à un changement de position de la personne pendant l'utilisation, et pour enregistrer et/ou transmettre une ou plusieurs instances du changement de position pour fournir des informations sur le changement de position de la personne sur le siège au dispositif électronique externe (42).

9. Système (40) pour une utilisation clinique dans la surveillance de l'activité d'une personne sur un coussin, un matelas ou une autre structure soutenant la personne en position assise ou allongée, le système (40) comprenant :
un dispositif de surveillance de pression (10) selon l'une quelconque des revendications précédentes ; et
un dispositif électronique externe (42) comprenant un moyen de communication (26) pour recevoir des données de pression du coussin de surveillance de pression, et un moyen de stockage de données pour stocker les données de pression,
dans lequel, en utilisation, le dispositif externe (42) établit une connexion avec le dispositif de surveillance de pression (10) et les données stockées dans l'enregistreur de données (25) sont transmises au dispositif électronique externe (42).

10. Système (40) selon la revendication 9, comprenant en outre un serveur (44), le dispositif externe (42), en utilisation, établissant une connexion au serveur (44) et transmettant les données de pression au serveur (44) par l'intermédiaire du moyen de communication (26) pour les stocker.

11. Système (40) selon la revendication 10, dans lequel, en utilisation, le serveur (44) traite les données de pression reçues pour produire des informations de pression et facultativement le serveur (44) transmet les informations de pression au dispositif externe (42).

12. Système (40) selon l'une quelconque des revendications 9 à 11, dans lequel une sortie graphique des données de pression est affichée sur le dispositif externe (42), la sortie graphique incluant un graphique montrant des états de pressostat pour indiquer l'utilisation du coussin, du matelas ou de la structure sur une période de temps.

13. Système (40) selon l'une quelconque des revendications 9 à 12, dans lequel une pluralité de dispositifs de surveillance de pression (10) sont prévus et, en utilisation, le dispositif externe (42) établit une connexion à un ou plusieurs parmi la pluralité de dispositifs de surveillance de pression (10).

14. Système (40) selon l'une quelconque des revendications 9 à 13, dans lequel le système (40) est configuré pour déterminer un changement de position de la personne sur le dispositif de surveillance de pression (10), et le dispositif externe (42) est configuré pour produire une notification de changement de position, facultativement dans lequel le changement de position est déterminé sur la base des données de pression sur une période de temps, dans lequel :
le dispositif de surveillance de pression (10) est configuré pour déterminer s'il y a eu un changement de position, et pour enregistrer et/ou transmettre une ou plusieurs instances du changement de position pour fournir des informations sur le changement de position de la personne sur le siège au dispositif électronique externe (42) ; ou
le dispositif externe (42) ou le serveur (44) est configuré pour déterminer s'il y a eu un changement de position.

15. Procédé de gestion du risque d'escarres par surveillance de l'activité d'une personne utilisant un siège, un matelas ou une autre structure soutenant la personne en position assise ou allongée, le procédé comprenant :
la connexion, par un moyen de communication sans fil (26), d'un dispositif externe (42) à un dispositif de surveillance de pression (10) selon l'une quelconque des revendications 1 à 8, le dispositif de surveillance de pression (10) étant disposé sur, dans ou sous le siège pour enregistrer des données de pression à l'aide d'un moyen de détection de pression configuré pour détecter la pression exercée par la personne ; et
la détermination, à l'aide de données de pression pendant une période de temps depuis que la personne a commencé à utiliser le siège ou depuis qu'un contrôle précédent de changement de position a été effectué, si la personne assise ou allongée sur le siège a sensiblement changé sa position assise ou allongée dans la période de temps en fournissant des informations au dispositif externe (42), ou en fournissant une notification sur le dispositif externe (42), liées aux données de pression à utiliser pour prendre une décision clinique ou évaluer si cette personne a sensiblement changé sa position assise ou allongée, par exemple pour lui demander de changer de position.
